Europäisches Patentamt

European Patent Office

Office européen des brevets

(11) Numéro de publication: **0 418 151 A1**

# (12) DEMANDE DE BREVET EUROPEEN

(21) Numéro de dépôt: 90402507.9

(22) Date de dépôt: 12.09.90

(51) Int. Cl.5: **C08L 1/24**, A61L 15/22, C08J 9/35

(30) Priorité: 12.09.89 FR 8911925

(43) Date de publication de la demande: 20.03.91 Bulletin 91/12

(84) Etats contractants désignés: BE DE ES FR GB IT NL

(71) Demandeur: FINANCIERE ELYSEES BALZAC
2, Rue Balzac
F-75008 Paris(FR)

(72) Inventeur: Colin, Claudine
3, Rue Henri Barbusse
F-60290 Rantigny(FR)
Inventeur: Jorda, Rafael
30, Rue Claude Jusseaud
F-69110 Ste Foy Les Lyons(FR)
Inventeur: Porte, Hugues
6, Chemin de Crépieux
F-69300 Caluire(FR)
Inventeur: Wertz, Jean-Luc
8, rue Sainte-Angadième
F-60000 Beauvais(FR)

(74) Mandataire: Le Roux, Martine et al
Cabinet Beau de Loménie 55, rue
d'Amsterdam
F-75008 Paris(FR)

(54) Matériau alvéolaire cellulosique à rétention d'eau améliorée et préparation de celui-ci.

(57) La présente invention a pour objet un matériau alvéolaire cellulosique, à rétention d'eau améliorée, caractérisé en ce qu'il contient des particules de polymère superabsorbant, piégées dans le réseau de cellulose. Elle concerne également un procédé de préparation d'un tel matériau.

EP 0 418 151 A1

## MATÉRIAU ALVÉOLAIRE CELLULOSIQUE À RÉTENTION D'EAU AMÉLIORÉE ET PRÉPARATION DE CELUI-CI.

La présente invention a pour objet un matériau alvéolaire cellulosique -ou éponge- à rétention d'eau (et plus généralement de liquides aqueux) améliorée. Elle concerne également un procédé de préparation d'un tel matériau.

La Demanderesse a mis au point un nouveau produit du type éponge, qui, outre le fait qu'il possède une capacité de rétention d'eau améliorée, reste humide et souple plus longtemps qu'une éponge de l'art antérieur. Son toucher et son aspect s'en trouvent améliorés.

En effet, à l'état sec, une éponge est rêche et se rétracte, alors qu'à l'état humide, elle a un toucher doux et agréable. Ce changement d'état se visualise particulièrement sur les éponges de faible épaisseur, qui à l'état sec, gondolent.

Pour remédier à ce problème -garder une éponge humide et souple, notamment lors du stockage ou de la vente- on a proposé, selon l'art antérieur, l'incorporation au sein de ladite éponge de chlorure de magnésium et de glycols. Cette solution ne se révèle pas pleinement satisfaisante. Ledit chlorure de magnésium et lesdits glycols s'éliminent dès les premières utilisations et ne jouent alors plus leur rôle de rétenteur d'eau et de plastifiant de la cellulose.

La Demanderesse propose, présentement, l'intervention de particules de polymère superabsorbant, dans la masse dudit matériau alvéolaire cellulosique. On fournit ci-après des précisions sur lesdites particules.

Des particules de polymère superabsorbant sont déjà, avantageusement, utilisées au sein de produits jetables, tels les couches culottes.

Leur intervention, selon l'invention, dans le domaine des éponges -à l'intérieur du réseau de cellulose- ne résulte pas d'une démarche évidente.

En effet, l'incorporation simple de particules de polymère superabsorbant au cours du procédé de fabrication des éponges ou matériaux alvéolaires cellulosiques -dit procédé viscose- sans protection desdites particules, entraîne inévitable ment une dégradation de celles-ci par hydrolyse et/ou écrasement et, par là même, une perte considérable de leur capacité de rétention d'eau.

L'addition desdites particules, après fabrication dudit matériau alvéolaire cellulosique, ne permet pas de les faire pénétrer à l'intérieur dudit matériau et de les y retenir mécaniquement. De plus, la présence desdites particules, à l'état gonflé après absorption d'eau, en surface dudit matériau ou de l'éponge, lui confère un toucher gluant, collant, guère approprié à son utilisation, notamment dans le domaine du nettoyage ménager.

On a décrit, dans la demande de brevet EP-A-0 293 208, des articles absorbants, obtenus par le procédé viscose et contenant des particules de polymère superabsorbant. Au sein de ces articles, lesdites particules ne sont pas piégées dans le réseau de cellulose. Lesdits articles sont généralement des matériaux stratifiés ; on trouve alors les particules de polymère superabsorbant entre deux couches de matériau cellulosique comprimé.

La Demanderesse propose présentement une méthode pour l'incorporation, sans dégradation et de façon stable, de particules de polymère superabsorbant dans la structure de matériaux alvéolaires cellulosiques ; afin d'obtenir les résultats escomptés, énoncés ci-dessus.

De tels matériaux, incluant lesdites particules dans leur structure cellulosique, constituent un objet de l'invention ; leur procédé de préparation en constitue un autre.

La présente invention a donc pour objet un matériau alvéolaire cellulosique, à rétention d'eau améliorée, caractérisé en ce qu'il contient des particules de polymère superabsorbant, piégées dans le réseau de cellulose.

De façon caractéristique, on trouve, dans les éponges de l'invention, les particules de polymère superabsorbant, piégées dans le réseau de cellulose. Elles sont localisées dans les alvéoles de ladite cellulose.

Une telle structure, nouvelle à ce jour, a pu être obtenue par le procédé décrit ci-après ; procédé viscose -connu de l'homme de métier- dans lequel on fait intervenir, selon l'invention, les particules de polymère superabsorbant, protégées temporairement, avant la régénération acide de la cellulose, au sein de la viscose.

Les particules de polymère superabsorbant assurent la fonction de rétenteurs d'eau ou de tout autre liquide. Il s'agit de particules de polymères superabsorbants (généralement connus sous le sigle S.A.P. : Super Absorbant Polymer).

Ces polymères peuvent être d'origine naturelle ou synthétique et sont avantageusement, aux fins de l'invention, insolubles dans l'eau.

Nous utiliserons parfois l'abréviation S.A.P., pour des raisons de commodité, dans la suite de la présente description.

Ces polymères -S.A.P.- mis en contact avec l'eau, forment un gel, physique ou chimique, appelé également hydrogel, capable d'absorber des quantités importantes d'eau dans son réseau macromoléculaire.

Des polymères superabsorbants (S.A.P.) d'origine naturelle sont par exemple des polymères dont le réseau macromoléculaire est de type polysaccharide tels que l'agar-agar, la gélose, l'alginate, la carboxyméthylcellulose, telle que décrite en particulier dans le brevet US-A-3 589 364, l'hydroxyéthylcellulose, la méthylcellulose, le triacétate de cellulose gélifié tel que décrit notamment dans les brevets US-A-1 693 890 et US-A-3 846 404, l'amidon greffé polyacrylonitrile tel que décrit notamment dans les brevets US-A-3 935 099 et US-A-3 661 815 et l'amidon greffé acide acrylique tel que décrit dans le brevet FR-A-2 305 452.

Des polymères superabsorbants (S.A.P.), d'origine synthétique sont par exemple, des polymères dont le réseau macromoléculaire est formé de polymères acryliques ; de polymères méthacrylate d'hydroxyéthyle ; d'alcool polyvinylique réticulé ; de polyacrylamide ; d'acétate de polyvinyle partiellement hydrolysé ; d'acrylate d'hydroxyéthyle ; de mono-acrylate de diéthylène-glycol ; de monométhacrylate de diéthylène-glycol ; d'acrylate de 2-hydroxypropyle; de méthacrylate de 2-hydroxypropyle ; d'acrylate de 3-hydroxypropyle ; de méthacrylate de 3-hydroxypropyle ; de monométhacrylate de dipropylène-glycol ; de vinylpyrrolidone ; d'acrylamide ; de méthacrylamide ; de N-propylacrylamide ; de N-isopropylméthacrylamide ; de N-méthylacrylamide ; de N-2-hydroxyéthylméthacrylamide ; d'hydrogels de polyuréthanne, formés de polymères légèrement réticulés de prépolymères terminés par un isocyanate qui sont les produits de réaction d'un poly(alkyléneoxy)polyol avec un diisocyanate organique légèrement réticulé avec de l'eau ou une polyamine organique, comme décrit dans le brevet US-A-3 939 105 ; de copolymères de monomères à insaturation éthylénique d'acrylates et de méthacrylates hydroxyalkyliques et d'acrylates et méthacrylates d'alcoxyalkylène-glycols, comme décrit dans le brevet US-A-4 038 264 ainsi que d'autres polymères superabsorbants (S.A.P.) connus de l'homme de l'art.

On préférera les polymères superabsorbants (S.A.P.) d'origine synthétique, insolubles dans l'eau, car ils sont plus stables thermiquement et présentent une moindre dégradation à l'action des microorganismes.

Les polymères superabsorbants (S.A.P.), insolubles dans l'eau, sont obtenus par réticulation de macromolécules et se présentent sous forme d'écailles, de poudres, de films, de fibres, de sphères ou "perles"...

Aux fins de l'invention, on utilisera de préférence des particules de forme sphérique, présentant un diamètre compris entre environ 50 et 400 μm.

Pour des raisons d'homogénéité de l'effet de rétention d'eau, on souhaitera que lesdites particules soient uniformément réparties et on utilisera avantageusement des particules de S.A.P., présentant un diamètre compris entre 50 et 200 μm.

On utilisera notamment des produits dits "perlés", obtenus par polymérisation en suspension inverse.

De tels produits sont, à ce jour, commercialisés en EUROPE par les sociétés HOECHST (Réf. PL 2255), BASF (Luquasorb HC 9797) et NORSOLOR (X 5 TS).

Pour l'obtention du résultat recherché, les matériaux alvéolaires cellulosiques selon l'invention contiennent généralement environ de 1 à 5 % en poids de particules de S.A.P. par rapport au poids de cellulose sèche. Il n'est nullement exclu qu'ils puissent en contenir une plus grande quantité, jusqu'à 10 % et plus. Il conviendra évidemment de vérifier que les quantités incorporées n'altèrent pas ou pas trop les propriétés du produit fini : toucher, aspect, tenue mécanique, ...

Selon une variante de l'invention, le matériau alvéolaire cellulosique est revêtu sur au moins une de ses faces d'un autre matériau. Il peut s'agir de tout type de matériau compatible avec le matériau alvéolaire cellulosique et dont l'intervention présente un quelconque intérêt dans le domaine d'utilisation dudit matériau alvéolaire cellulosique. On citera, à titre illustratif et nullement limitatif, l'intervention d'une mousse expansée de latex, réticulée du type notamment de celle constituant la face essuyante du produit décrit dans la demande de brevet EP 88 401 930.8 de la Demanderesse, publiée sous le numéro 0 335 050.

On a constaté que l'intervention d'une telle mousse en latex synthétique améliore encore le pouvoir rétenteur d'eau du matériau alvéolaire cellulosique auquel ladite mousse est associée. La cinétique d'évaporation de l'eau s'en trouve diminuée sans altération aucune des propriétés du produit type éponge présentant au moins une face essuyante en latex synthétique.

Avantageusement, un tel matériau composite contient également des particules de polymère superabsorbant entre ledit matériau alvéolaire cellulosique et ledit autre matériau.

On rencontre donc, au sein de ce matériau composite, des particules de polymère superabsorbant à l'interface matériau alvéolaire cellulosique / autre matériau et dans la masse dudit matériau alvéolaire

3

cellulosique.

Comme précisé ci-dessus, les matériaux alvéolaires cellulosiques de l'invention doivent leurs propriétés intéressantes au fait qu'ils renferment des particules de S.A.P., non dégradées au sein de leur réseau cellulosique.

La méthode d'incorporation desdites particules au sein desdits matériaux constitue donc un élément clef de la présente invention. Cette incorporation a lieu en cours de préparation dudit matériau, par le procédé viscose, connu de l'homme de l'art.

Ce procédé pour l'élaboration de matériaux alvéolaires cellulosiques est résumé ci-après.

De la pâte de bois est utilisée comme matière première. Elle est mise en solution par le procédé viscose en passant par le dérivé xanthate avec adjonction d'agent porophore et de fibres. La pâte obtenue peut être enduite, par exemple de part et d'autre d'une grille. Cette étape d'enduction est suivie d'une étape de régénération acide de la cellulose. La pâte peut aussi être introduite dans un moule puis régénérée en milieu basique. La cellulose ainsi régénérée est alors rincée, soumise à un traitement de blanchiment, découpée et emballée. Ce procédé peut éventuellement comporter une ou plusieurs étapes de modification du produit fini, et notamment le revêtement d'au moins une des faces dudit produit par un autre matériau.

On élabore le matériau alvéolaire cellulosique de l'invention dans le cadre du procédé viscose en y ajoutant les étapes supplémentaires :
- d'incorporation de particules de polymère superabsorbant, protégées temporairement dans le xanthate de cellulose (viscose) avant régénération,
- éventuellement, de saupoudrage de particules de polymère superabsorbant protégées temporairement sur au moins une face dudit matériau alvéolaire cellulosique obtenu avec régénération acide ou basique de la cellulose, avant le revêtement de ladite face par un autre matériau
- et de déprotection, en phase finale de fabrication du matériau, desdites particules de polymère superabsorbant.

Selon une première variante, lesdites particules de S.A.P. sont introduites dans le xanthate de cellulose (viscose), de préférence, après l'adjonction du porophore et des fibres.

Dans cette hypothèse, elles sont incorporées, soit dans le malaxeur, assurant le mélange dudit porophore, des fibres et du dérivé xanthate, soit lors de l'enduction du mélange obtenu -dérivé xanthate (viscose) + fibres + porophore- et de préférence, entre deux couches dudit mélange.

Lesdites particules de polymère superabsorbant interviennent donc dans le procédé, protégées temporairement. Leur protection doit être telle que d'une part, elles supportent sans dommage les étapes du procédé viscose qu'elles ont éventuellement à subir et/ou les étapes supplémentaires de modification du produit obtenu par ledit procédé viscose et que, d'autre part, on puisse s'en débarrasser sans altération desdites particules, de leur propriétés et du matériau dans lequel elles sont présentes.

Une telle protection peut leur être assurée par encapsulation dans une membrane monocouche ou bicouche, consistant essentiellement en un (ou deux) mélange(s) d'au moins un polymère filmogène et d'au moins un matériau cristallin hydrophobe.

Une telle membrane fait l'objet d'une demande de brevet français au nom de la Demanderesse, déposée le 12 septembre 1989 sous le n° 89 11 924.

L'association de ces deux types de matériaux -polymère(s) filmogène(s), matériau(x) cristallin(s) hydrophobe(s)- permet l'élaboration d'une membrane étanche dans les conditions sévères du procédé viscose ; membrane qui toutefois autorise la libération du S.A.P. encapsulé. La dégradation ou l'élimination de ladite membrane, pour la libération du S.A.P., peut être réalisée notamment par voie mécanique (écrasement, broyage, ultrasons), par voie thermique (chauffage, congélation), par rayonnement actinique (U.V., I.R., faisceaux d'électrons, micro-ondes) et par voie chimique (variation de pH, mise en solution, attaque chimique). Généralement, on libère ledit S.A.P., par fusion du ou des matériaux cristallins hydrophobes, intervenant dans la composition de sa membrane.

Avantageusement, lesdits matériaux de la membrane sont des composés organosolubles. Leur solubilité dans les solvants organiques permet la mise en oeuvre de techniques particulièrement performantes pour l'enrobage des particules de polymère superabsorbant (S.A.P.) ; techniques qui seront précisées ci-après.

Toutefois, on ne saurait, selon l'invention, exclure l'emploi de composés peu ou pas organosolubles, emploi associé à la mise en oeuvre d'autres techniques d'enrobage ou de pelliculage.

Le polymère filmogène intervenant dans la composition de la membrane selon l'invention peut être choisi parmi les polymères d'origine naturelle, les polymères d'origine synthétique ou leurs mélanges.

On peut avantageusement le choisir parmi les dérivés cellulosiques -tels que les éthylcelluloses, les acétophtalates ou les acétobutyrates de cellulose- ou leurs mélanges.

De la même façon, on peut avantageusement le choisir parmi les (co)polymères de styrène, les (co)-polymères à base de chlorure de vinylidène... ou leurs mélanges.

Comme indiqué ci-dessus, il peut même consister en un mélange d'au moins un polymère d'origine naturelle et d'au moins un polymère d'origine synthétique.

Le matériau cristallin hydrophobe intervenant dans la composition de la membrane est avantageusement choisi parmi les acides gras, les dérivés d'acides gras (amides ou bistéaramides d'acides gras...), les cires microcristallines et leurs mélanges. On peut notamment utiliser l'acide béhénique, l'acide stéarique, le stéaramide, le bistéaramide ou l'érucamide. On choisit avantageusement un matériau cristallin "pur", présentant un point de fusion franc. Il est alors toujours possible, pour libérer la particule de polymère superabsorbant (S.A.P.) encapsulée, d'élever la température du milieu -matériau alvéolaire cellulosique-dans lequel elle se trouve au moins et éventuellement au-dessus dudit point de fusion franc.

Les deux principaux constituants de la membrane interviennent avantageusement dans les proportions pondérales suivantes : environ 10 à 90 % de polymère filmogène pour environ 90 à 10 % de matériau cristallin hydrophobe.

Un mélange, particulièrement préféré, notamment lorsqu'un polymère d'origine naturelle intervient, comprend environ 30 % dudit polymère filmogène et 70 % de matériau cristallin hydrophobe.

La membrane peut contenir, outre ses deux constituants principaux, des additifs et notamment des produits destinés à améliorer son mouillage et\ou son hydrophobie et/ou son adhésion sur la particule de polymère superabsorbant (S.A.P.). Par exemple, on peut ajouter des charges lamellaires minérales comme le mica pour améliorer l'hydrophobie et l'étanchéité à l'eau de la membrane.

Selon une des variantes préférées de l'invention, on fait intervenir des particules de S.A.P. encapsulées dans une membrane monocouche, consistant essentiellement en un mélange d'environ 30 % en poids de polystrène et d'environ 70 % en poids de stéaramide.

Selon une autre variante préférée de l'invention, on fait intervenir des particules de S.A.P. encapsulées dans une membrane bicouche dont la première couche consiste essentiellement en un mèlange d'environ 30 % en poids de polystrène et d'environ 70 % en poids de stéaramide et dont la seconde couche, couche au contact de la particule S.A.P., consiste essentiellement en un mélange d'environ 30 % en poids d'éthylcellulose et d'environ 70 % en poids d'acide béhénique.

Il est évident que la nature des constituants intervenant dans la composition de la membrane et les proportions dans lesquelles ils interviennent jouent un rôle important sur l'efficacité et la durée de la protection du S.A.P. encapsulé. D'autres facteurs entrent également en ligne de compte et notamment l'épaisseur de ladite membrane ainsi que le procédé mis en jeu pour sa constitution.

L'homme de l'art est à même de déterminer chacun desdits paramètres pour l'optimisation de ladite protection.

En ce qui concerne l'épaisseur de la membrane, il est évident qu'en l'augmentant, on améliore la protection du produit (S.A.P.) encapsulé.

En général, une protection efficace -au cours de la préparation des matériaux alvéolaires cellulosiques de l'invention-du S.A.P. encapsulé est obtenue avec une épaisseur de membrane comprise entre 10 et 50 µm.

L'épaisseur de la membrane monocouche ou bicouche dont la nature des constituants a été précisée ci-dessus est avantageusement comprise entre 25 et 40 µm.

Pour l'encapsulation des particules de S.A.P., on peut mettre en oeuvre toutes les techniques d'encapsulation connues adaptées.

Avantageusement, ledit S.A.P. peut être encapsulé par pulvérisation des matériaux, appelés à constituer la ou les membranes successives, lesdits matériaux étant solubilisés dans un solvant convenable. Ce procédé peut notamment être mis en oeuvre avec des matériaux organosolubles, en solution dans un solvant organique, qui ne fait pas gonfler le polymère superabsorbant (S.A.P.) à encapsuler. Des solvants usuels, tels que les alcools, les solvants chlorés, les alcanes... ou leurs mélanges peuvent être utilisés.

Ce procédé est avantageusement mis en oeuvre pour générer une encapsulation par pelliculage, notamment en lit fluidisé. Des techniques telles que celles des procédés Wurster, Top Spray ou pulvérisation tangentielle peuvent être mises en oeuvre. Ces procédés permettent un pelliculage successif de la particule de polymère superabsorbant au cours des multiples passages de celle-ci près de la buse de pulvérisation.

D'autres techniques d'enrobage, connues de l'homme de l'art, permettent l'obtention des particules de S.A.P. aux fins de l'invention. On citera notamment les techniques par fusion.

L'étanchéité des particules enrobées obtenues peut encore, dans certains cas, être améliorée par un traitement thermique ou recuit (à une température inférieure au point de fusion du matériau cristallin hydrophobe).

Un tel traitement thermique peut être effectué après l'enrobage par une unique couche ; ou après l'enrobage par une première couche et/ou après l'enrobage par une seconde couche.

Les particules ainsi encapsulées gardent intactes leur capacité de rétention d'eau. On a pu constater que même si le passage de la particule dans différents milieux réactionnels fragilisait sa coque protectrice, la protection demeurait efficace.

Le problème de la déprotection desdites particules se pose à l'issue du procédé de fabrication du matériau.

Avantageusement, ladite déprotection se réalise au cours d'une phase terminale de fabrication dudit matériau.

Cette déprotection, notamment lorsqu'elle consiste en l'élimination de la membrane monocouche ou bicouche décrite ci-dessus peut être réalisée par voie mécanique, par voie thermique ou par rayonnement actinique et voie chimique.

La déprotection par fusion (voie thermique) est préférée. A cette fin, on peut traiter thermiquement le produit obtenu soit dans un tunnel à air chaud, soit par de la vapeur d'eau chaude, soit par micro-ondes ou hautes fréquences... Pour l'obtention du résultat recherché -élimination de la membrane décrite ci-dessus-, on traite généralement le produit à au moins 100°C pendant quelques secondes.

On notera qu'avantageusement, ladite déprotection peut être obtenue simultanément à la réticulation d'un matériau intervenant sur au moins une face du matériau alvéolaire cellulosique. On a vu que de tels matériaux pouvaient consister en une mousse expansée de latex.

Le matériau alvéolaire cellulosique de l'invention peut donc être obtenu par le procédé viscose avec intervention des particules de S.A.P., protégées temporairement.

Cette protection peut être assurée par la membrane monocouche ou bicouche décrite ci-dessus.

D'autres types de protection peuvent être mis au point et ne sauraient être exclus du cadre de la présente invention.

La Demanderesse précise ci-après deux variantes préférées de la méthode de préparation de matériaux alvéolaires cellulosiques, selon l'invention.

Selon la première variante, on prépare des éponges dans la masse desquelles des particules de S.A.P. sont de préférence uniformément réparties.

Le procédé viscose est mis en oeuvre. Les particules de S.A.P., encapsulées, sont incorporées dans le xanthate de cellulose (viscose). Le produit obtenu subit une régénération acide. Après rinçage et blanchiment, on soumet à un traitement thermique le matériau alvéolaire cellulosique obtenu.

Ledit traitement thermique a pour but de libérer lesdites particules de S.A.P., réparties dans la masse dudit matériau. Il est réalisé à au moins 100°C pendant quelques secondes.

Selon la deuxième variante, on prépare un matériau alvéolaire cellulosique essuyant, tel que décrit dans la demande EP 0 335 050 de la Demanderesse.

Sur une face d'un matériau alvéolaire cellulosique obtenu par le procédé viscose, tel que modifié ci-dessus (par incorporation de particules de S.A.P. encapsulées, dans le xanthane de cellulose), on saupoudre des particules de S.A.P. encapsulées. Ladite face est alors enduite d'une mousse de latex non réticulée. L'ensemble est ensuite soumis à un traitement thermique dont les paramètres sont fixés pour assurer à la fois la réticulation de ladite mousse et la libération des particules de S.A.P. encapsulées. La détermination de ces paramètres est à la portée de l'homme de métier.

Les exemples ci-après illustrent l'invention et en confirment l'intérêt.

Exemple 1 (art antérieur)

Cet exemple est donné à titre de référence.

| | Fabrication d'une éponge standard sans addition de S.A.P. | |
|---|---|---|
| a) | Pâte de bois | |
| | ↓ | NaOH |
| b) | Alcali-cellulose | |
| | ↓ | $CS_2$ |
| c) | Xanthate de cellulose (viscose) | |
| | ↓ | Ajout de fibres et de porophore |
| d) | Pâte | |
| | ↓ | ←νφξψτ ον φε ραθτ ετ φ΄αξτθε φ΄ξνε λθ ωωε Bains acides |
| e) | Cellulose régénérée | |
| | ↓ | Rinçage - blanchiment Incorporation de $MgCl_2$ Découpe - Emballage |
| f) | Produit fini (290 g/m² de cellulose sèche) | |
| . | Dimensions | 20 x 20 cm |
| . | Epaisseur | 6 mm |
| . | Poids de cellulose | 10 g |
| . | Poids de chlorure de magnésium $MgCl_2$ | 3 g |
| . | Poids d'eau (g) | 15 g |

Exemple 2

| Fabrication d'une éponge selon l'invention contenant 5 % de S.A.P. dans sa masse. | | |
|---|---|---|
| a) | Pâte de bois | |
| | ↓ | NaOH |
| b) | Alcali cellulose | |
| | ↓ | $CS_2$ |
| c) | Xanthate de cellulose (viscose) | |
| | ↓ | Fibres + Porophore |
| d) | Pâte | |
| | ↓ | ←νφξψτ ον φε ρ=τε ετ σαξροξφθαλε φε Στ∇τℓτ ενψαρσξω σ εν σανφδ ψη εντθε φεξχ ψοξψησσ φε Χ σψοσε<br>+<br>Bain acide |
| e) | Cellulose régénérée | |
| | ↓ | Rinçage - Blanchiment<br>Traitement thermique (100°C pendant 10 secondes)<br>Découpe emballage |
| f) | Produit fini | |
| . | Dimensions | 20 x 20 cm |
| . | Epaisseur | 6 mm |
| . | Poids de cellulose | 10 g |
| . | Poids de S.A.P. | 0,5 g |
| . | Poids d'eau | 20 g |

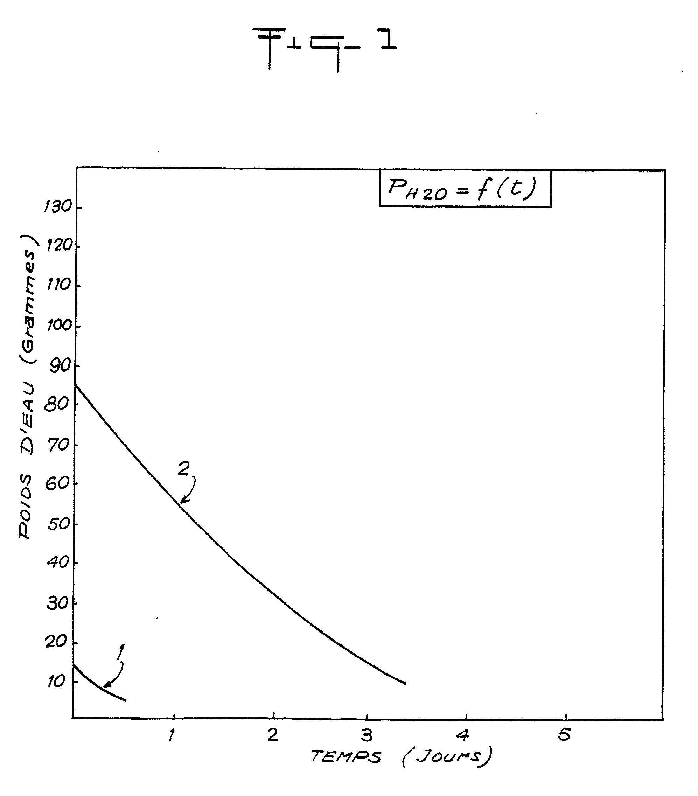

Caractéristiques des particules de S.A.P. intervenant :
- Diamètre des S.A.P. 280 - 400 $\mu$m
- Enrobage bicouche :
couche interne : acide béhénique - éthylcellulose (70/30)
couche externe : stéaramide - polystyrène (70/30)
- Epaisseur de l'enrobage 30 $\mu$m
Les échantillons des exemples 1 à 2 ont été testés.
Le tableau 1 ci-après et la figure 1 jointe à la présente illustrent l'impact des modifications apportées selon l'invention sur les caractéristiques d'absorption et de rétention d'eau des produits ainsi que sur leur cinétique d'évaporation d'eau.

Tableau 1

| Absorption et rétention d'eau de ville des échantillons 1 à 2 | | |
|---|---|---|
| | (1) Absorption d'eau (g) | (2) Rétention d'eau (g) |
| Echantillon ex 1 | 100 - 120 | 10 - 15 |
| Echantillon ex 2 | 175 - 200 | 80 - 90 |

(1) Absorption totale d'eau de ville mesurée selon la norme Edana 10.1-72

(2) Rétention d'eau de ville après centrifugation (3000 trs.mn 12 secondes). La centrifugation correspond à un exprimage manuel moyen de l'eau absorbée par le produit.

Les courbes d'évaporation d'eau (figure 1) ont été établies en atmosphère contrôlée : 20° C - 50 % H.R. Elles donnent la cinétique d'évaporation de l'eau des échantillons après un rinçage à l'eau de ville suivi d'une centrifugation dans les conditions (2) reprises ci-dessus.

Le tableau 1 et la figure 1 démontrent l'amélioration de la rétention d'eau recherchée.

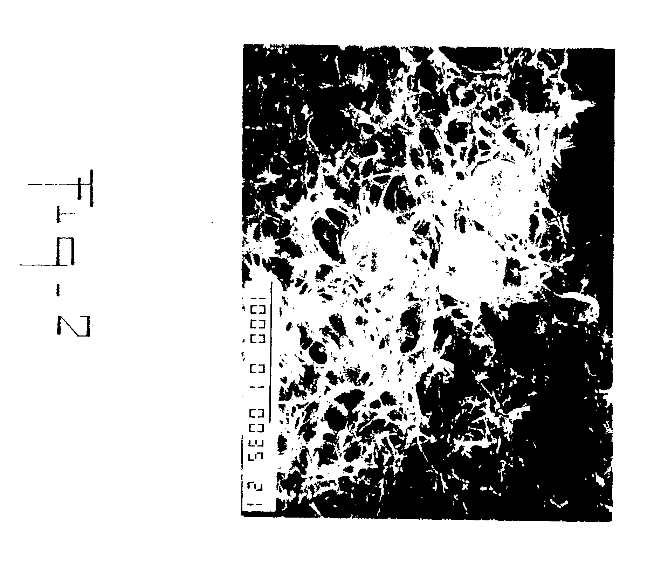

Sur la figure 2 -cliché en coupe d'une toile éponge de l'invention, cliché cbtenu par microscopie électronique à balayage (grossissement x 35)- on voit clairement que les particules de polymère superabsorbant sont à l'intérieur des alvéoles de la cellulose (dans le réseau de cellulose).

## Revendications

1. Matériau alvéolaire cellulosique, à rétention d'eau améliorée, caractérisé en ce qu'il contient des particules de polymère superabsorbant, piégées dans le réseau de cellulose.

2. Matériau alvéolaire cellulosique, selon la revendication 1, caractérisé en ce que lesdites particules sont sphériques, d'un diamètre d'environ 50 à 400 μm, de préférence compris entre 50 et 200 μm.

3. Matériau alvéolaire cellulosique selon l'une des revendications 1 ou 2, caractérisé en ce qu'il contient environ 1 à 5 % en poids desdites particules par rapport au poids de cellulose sèche.

4. Matériau alvéolaire cellulosique selon l'une quelconque des revendications 1 à 3, caractérisé en ce qu'il est revêtu sur au moins une de ses faces d'un autre matériau, tel une mousse expansée de latex, réticulée.

5. Matériau alvéolaire cellulosique selon la revendication 4, caractérisé en ce qu'il contient en outre des particules de polymère superabsorbant entre ledit matériau alvéolaire cellulosique et ledit autre matériau.

6. Préparation par le procédé viscose d'un matériau alvéolaire cellulosique selon l'une quelconque des revendications précédentes -à partir de pate de bois mise en solution, en passant par le dérivé xanthate (viscose), avec adjonction de porophore et de fibres, régénération acide ou basique de la cellulose obtenue, rinçages et blanchiment- suivie éventuellement d'un revêtement d'au moins une de ses faces par un autre matériau, caractérisé en ce qu'elle comporte les étapes supplémentaires :

- d'incorporation de particules de polymère superabsorbant, protégées temporairement dans le xanthate de cellulose (viscose) avant régénération,

- éventuellement, de saupoudrage de particules de polymère superabsorbant, protégées temporairement sur au moins une face dudit matériau alvéolaire cellulosique obtenu avec régénération acide ou basique de la cellulose, avant le revêtement de ladite face par un autre matériau,

- et de déprotection, en phase finale de fabrication du matériau, desdites particules de polymère superabsorbant.

7. Préparation selon la revendication 6, caractérisée en ce que des particules de polymère superabsorbant, protégées temporairement, sont incorporées après l'adjonction du porophore et des fibres, soit dans le malaxeur assurant le mélange dudit porophore, des fibres et du dérivé xanthate (viscose), soit lors de l'enduction du mélange obtenu -dérivé xanthate (viscose) + fibres + porophore- et de préférence, entre deux couches dudit mélange.

8. Préparation selon l'une des revendications 6 ou 7, caractérisée en ce que lesdites particules de polymère superabsorbant, incorporées et éventuellement saupoudrées, sont enrobées dans une membrane monocouche ou bicouche, consistant essentiellement en un (ou deux) mélange(s) d'au moins un polymère filmogène et d'au moins un matériau cristallin hydrophobe.

9. Préparation selon la revendication 8, caractérisée en ce que ledit polymère filmogène est choisi parmi :

- les polymères d'origine naturelle tels que les dérivés cellulosiques et notamment les éthylcelluloses, les acétophtalates de cellulose ou les acétobutyrates de cellulose,
- les polymères d'origine synthétique tels que les (co)polymères de styrène ou à base de chlorure de vinylidène,
- ou leurs mélanges,

et en ce que ledit matériau cristallin est choisi parmi les acides gras -et notamment l'acide béhénique, l'acide stéarique-, les dérivés d'acide gras -et notamment le stéaramide, le bistéaramide ou l'érucamide-, les cires microcristallines et leurs mélanges.

10. Préparation selon l'une des revendications 8 ou 9, caractérisée en ce que lesdites particules de polymère superabsorbant sont enrobées dans une membrane monocouche, consistant essentiellement en un mélange d'environ 30 % en poids de polystyrène et d'environ 70 % en poids de stéaramide.

11. Préparation selon l'une des revendications 8 ou 9, caractérisée en ce que lesdites particules de polymère superabsorbant sont enrobées d'une membrane bicouche, dont la première couche (couche externe) consiste essentiellement en un mélange d'environ 30 % en poids de polystyrène et d'environ 70 % en poids de stéaramide et dont la seconde couche consiste essentiellement en un mélange d'environ 30 % en poids d'éthylcellulose et d'environ 70 % en poids d'acide béhénique.

12. Préparation selon l'une quelconque des revendications 8 à 11, caractérisée en ce que lesdites particules de polymère superabsorbant sont déprotégées, à l'issue du procédé de fabrication du matériau ou au cours d'une phase terminale de fabrication de celui-ci, par dégradation voire élimination de la membrane par voie mécanique, thermique, chimique ou par rayonnement actinique.

13. Préparation selon l'une quelconque des revendications 8 à 12, caractérisée en ce que lesdites particules' de polymère superabsorbant encapsulé sont incorporées dans le xanthate de cellulose (viscose) et en ce qu'après régénération acide, le matériau est notamment rincé, blanchi et soumis à un traitement thermique -de quelques secondes, à plus de 100 °C- pour la libération desdites particules de polymère superabsorbant au sein du matériau alvéolaire cellulosique obtenu.

14. Préparation selon l'une quelconque des revendications 8 à 12, caractérisée en ce que des particules de polymère superabsorbant encapsulées sont saupoudrées sur une face du matériau alvéolaire cellulosique obtenu par le procédé viscose avec incorporation de particules de polymère superabsorbant encapsulées dans le xanthate de cellulose (viscose), ladite face étant ensuite enduite d'une mousse de latex non réticulée et soumise à un traitement thermique assurant à la fois la réticulation de ladite mousse et la libération des particules de polymère superabsorbant.

Fig_2

**Office européen
des brevets**

## RAPPORT DE RECHERCHE
### EUROPEENNE

Numéro de la demande

**EP 90 40 2507**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 293 208   (LION CORP.)<br>* Page 3, lignes 29-40; revendications *<br>− − − | 1-7 | C 08 L 1/24<br>A 61 L 15/22<br>C 08 J 9/35 |
| Y | EP-A-0 055 056   (ROHM AND HAAS CO.)<br>* Exemple II; revendications; page 6, lignes 15-19 *<br>− − − | 1-7 | |
| A | US-A-4 429 001   (B.E. KOLPIN et al.)<br>− − − | | |
| A | US-A-3 954 493   (O.A. BATTISTA et al.)<br>− − − | | |
| A | US-A-4 169 121   (H. PIETSCH et al.)<br>− − − | | |
| A | FR-A-2 380 783   (HOECHST)<br>− − − | | |
| A | US-A-4 069 366   (C.E. HOEY)<br>− − − | | |
| D,A | EP-A-0 335 050   (SPONTEX)<br>& AU-D-88 20 237 (07-09-1989)<br>− − − − − | | |

|  | DOMAINES TECHNIQUES RECHERCHES (Int. Cl.5) |
|---|---|
|  | C 08 L<br>C 08 J<br>A 61 L |

Le présent rapport de recherche a été établi pour toutes les revendications

| Lieu de la recherche | Date d'achèvement de la recherche | Examinateur |
|---|---|---|
| La Haye | 14 décembre 90 | LENSEN H.W.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

& : membre de la même famille, document correspondant